# EUROPEAN PATENT SPECIFICATION

(11) **EP 3 400 875 B1**
(45) Date of publication and mention of the grant of the patent: **28.02.2024**
(21) Application number: 18169578.4
(22) Date of filing: 26.04.2018
(51) Int. Cl.: G16H 20/10, G16H 20/40, A61B 6/00, A61B 10/02

(54) **SYSTEM AND METHOD FOR MONITORING AN AMOUNT OF CONTRAST AGENT WITHIN AN OBJECT**
SYSTEM UND VERFAHREN ZUR ÜBERWACHUNG EINER MENGE EINES KONTRASTMITTELS IN EINEM OBJEKT
SYSTÈME ET PROCÉDÉ DE SURVEILLANCE D'UNE QUANTITÉ D'AGENT DE CONTRASTE À L'INTÉRIEUR D'UN OBJET

(30) Priority: 28.04.2017 US 201715581384
(43) Date of publication of application: 14.11.2018
(73) Proprietor: General Electric Company, Schenectady, NY 12345 (US)
(72) Inventor: MILIONI DE CARVALHO, Pablo, 75013 Paris (FR); CARTON, Ann-Katherine, 92140 Clamart (FR); PALMA, Giovanni, 92130 Issy-Les-Moulineaux (FR); IORDACHE, Razvan, 75010 Paris (FR); MULLER, Serge, 78280 Guyancourt (FR)
(74) Representative: Fennell, Gareth Charles

(56) References cited:
- WO-A1-2014/168206
- US-A1- 2006 235 297
- US-A1- 2010 292 570
- US-A1- 2011 313 287
- US-A1- 2015 250 437
- KYONGTAE T. BAE: "Intravenous Contrast Medium Administration and Scan Timing at CT: Considerations and Approaches 1", RADIOLOGY, vol. 256, no. 1, 1 July 2010 (2010-07-01), pages 32-61, XP055291715, US ISSN: 0033-8419, DOI: 10.1148/radiol.10090908

## Description

Embodiments of the invention relate generally to medical imaging systems, and more specifically, to a system and method for monitoring an amount of a contrast agent within an object.

Contrast agents are chemical substances injected into an object/patient in order to improve the contrast in images of the object obtained by an imaging device/system. For example, contrast agents are used in many x-ray imaging procedures such as contrast-enhanced spectral mammography ("CESM"). Many contrast agents must be present within a region of interest ("ROI") of the patient at amounts higher than a minimum threshold in order to be effective. In many situations, however, the contrast agent is typically filtered out of the patient by one or more organs, e.g., kidneys, which lowers the total amount of the contrast agent within the ROI over time, and in turn lowers the contrast in subsequent obtained images. The presence of too much contrast agent within a patient, however, may cause one or more organs, e.g., the kidneys, to fail, and/or cause the patient to experience an allergic reaction. Thus, controlling the amount of a contrast agent within an ROI may be seen as a balancing act in which a physician must ensure that enough contrast agent is present within the ROI to maintain image quality, while also ensuring that the amount of the contrast agent within the patient remains low enough to reduce the risk of organ failure.

Accordingly, a contrast agent is usually manually reinjected into a patient several times during a medical imaging procedure based on a predetermined time schedule. Such time schedules are typically derived prior to the start of a medical procedure from a static model of the diffusion rate of the contrast agent within the patient. Due to environmental variances, e.g., changes in a patient's blood pressure, respiratory patterns, bodily movements, etc., the actual diffusion rate of the contrast agent within the object often varies significantly from the one predicted by the static model. Thus, medical practitioners may inject either too much or too little contrast agent into a patient while performing a medical imaging procedure.

US 2015/0250437 A1 describes an X-ray diagnostic apparatus including a processing circuitry.

WO 2014/168206 A1 describes a setting-screen display unit that makes a display unit display information regarding a base pattern for setting an injection protocol.

Kyongtae T. Bae: "Intravenous Contrast Medium Administration and Scan Timing at CT: Considerations and Approaches", Radiology, vol. 256, no. 1, 1 July 2010, pages 32-61, US describes intravenous contrast medium administration and scan timing at CT.

What is needed, therefore, is an improved system and method for monitoring an amount of a contrast agent within an object.

Aspects of the invention are defined in the independent claims.

The present invention will be better understood from reading the following description of non-limiting embodiments, with reference to the attached drawings, wherein below:
FIG. 1 is a block diagram of a system for monitoring an amount of a contrast agent within an object, in accordance with an embodiment of the present invention;
FIG. 2 is a diagram of an example of a medical workflow for an imaging procedure which utilizes the system of FIG. 1, in accordance with an embodiment of the present invention;
FIG. 3 is a diagram of a kinetic model of the system of FIG. 1, in accordance with an embodiment of the present invention;
FIG. 4 is another diagram of the kinetic model of the system of FIG. 1, in accordance with an embodiment of the present invention; and
FIG. 5 is a flow chart depicting a method for monitoring the amount of the contrast agent within the object utilizing the system of FIG. 1, in accordance with an embodiment of the present invention.

Reference will be made below in detail to exemplary embodiments of the invention, examples of which are illustrated in the accompanying drawings. Wherever possible, the same reference characters used throughout the drawings refer to the same or like parts, without duplicative description.

As used herein, the terms "substantially," "generally," and "about" indicate conditions within reasonably achievable manufacturing and assembly tolerances, relative to ideal desired conditions suitable for achieving the functional purpose of a component or assembly. As used herein, "electrically coupled," "electrically connected," and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present. The term "real-time," as used herein, means a level of processing responsiveness that a user senses as sufficiently immediate or that enables the processor to keep up with an external process. As further used herein, the terms "imaging procedure" and/or "medical imaging procedure" refer to a medical procedure that involves an imaging system to assist in accomplishing one or more tasks. Accordingly, as also used herein, the term "task" means an objective of a medical procedure, e.g., obtaining a biopsy, deploying/installing a stent into a blood vessel, locating an ulcer, imaging a clogged artery, suturing a patient, and/or other medical processes.

Additionally, while the embodiments disclosed herein are described with respect to an x-ray based imaging system, it is to be understood that embodiments of the present invention are equally applicable to other devices such as Magnetic Resonance Imaging ("MRI") systems, Positron Emission Tomography ("PET"), real-time endoscopic imaging, and/or any other type of imaging system that utilizes a contrast agent. As will be appreciated, embodiments of the present invention related imaging systems may be used to analyze objects within any material which can be internally imaged, generally. As such, embodiments of the present invention are not limited to analyzing objects within human tissue.

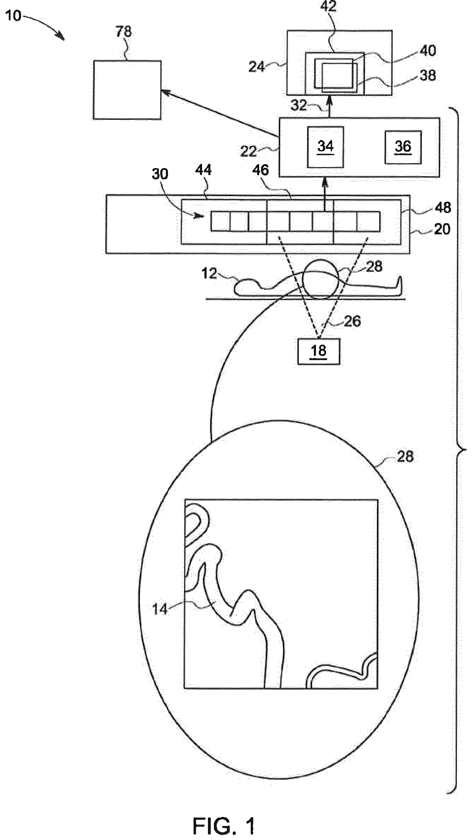

Referring now to FIG. 1, a system 10 for monitoring an amount of a contrast agent, e.g., iodine, within an object/patient 12, in accordance with embodiments of the invention, is shown. As will be understood, the system 10 is operative to image a structure 14, e.g., an internal organ, blood vessel, etc., within the patient 12. For example, the patient 12 may be undergoing a breast biopsy procedure, and the imaged structure 14 may be a lesion within one of the patient's 12 breasts. As shown in FIG. 1, the system 10 includes: a radiation source 18 and a detector 20, which collectively form an imaging device; a controller 22; and a display screen 24. The radiation source 18 projects a radiation beam 26 through an ROI 28 of the patient 12 within which the structure 14 is disposed. The radiation beam 26 is received by the detector 20, which generates a plurality of images 30 that are then communicated to the controller 22, which generates a video feed 32 that is transmitted to and displayed by the display screen 24.

As further shown in FIG. 1, the controller 22 includes at least one processor/CPU 34 and at least one memory device 36, and is in electronic communication with the radiation source 18, detector 20, and/or the display screen 24. An imaging program/application may be stored in the at least one memory device 36 that, when loaded into the at least one processor 34, adapts the controller 22 to generate the video feed 32 by processing the images 30 received from the detector 20. In embodiments, the imaging program may further adapt the controller 22 to control the detector 20 and/or the radiation source 18.

The video feed 32 includes a plurality of frames 38, 40, and 42. As used herein, the term frame describes a composite image that may be based at least in part on one or more of the plurality of images 30 acquired by the system 10. For instance, in embodiments, a single composite image/frame 42 may be generated by registering one or more of the acquired images 30 to a reference image selected from the plurality of images 30. The registration of one or more images 30 to a reference image may increase the contrast of the structure 14 within the produced/generated frame 42. Accordingly, in embodiments, each frame 38, 40, and 42 may be based at least in part on one or more of the images 30 received by the controller 22 from the detector 20. Once a frame 42 has been generated, it is transmitted, as part of the video feed 32, by the controller 22 to the display screen 24. In other words, in embodiments, the displayed video feed 32 is a processed form of the raw images 30 acquired by the system 10. In embodiments, the video feed 32 may be a live/real-time and/or near-real-time feed. In other embodiments, one or more of the frames 38, 40, and 42 may be still images, e.g., a photograph.

As will be understood, the system 10 may acquire one or more images 30 as part of an image acquisition 44, 46, 48, wherein the images 30 within the same acquisition 44, 46, 48 are acquired between injections of the contrast agent into the patient 12.

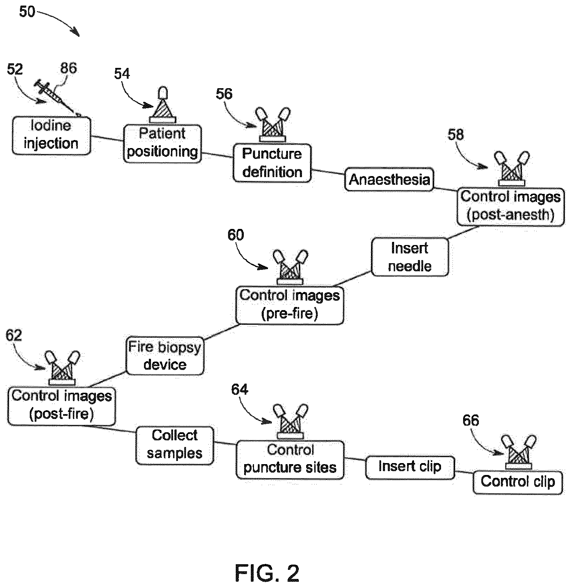

As illustrated in FIG. 2, the imaging device 18, 20 may be utilized to image the ROI 28 as part of a medical imaging procedure 50, e.g., a breast biopsy. As such, the patient 12 maybe given a first injection 52 of the contrast agent at the ROI 28 and subsequently imaged 54, 56, 58, 60, 62, 64, and 66 via the imaging device 18, 20. As the amount of the contrast agent at the ROI 28 degrades over time, embodiments of the invention may monitor/obtain contrast data from one or more of the images 30 obtained via the imaging device 18, 20. As will be appreciated, the term "contrast data," as used herein, refers to data acquired from the images 30 that corresponds to the contrast agent, e.g., provides an indication of the amount of contrast within the object/patient 12. Similarly, the term "contrast signal," as used herein, refers to the medium through which the contrast data is conveyed. For example, in embodiments, the contrast signal may be a grayscale scheme where black and white represents high and low amounts of the contrast agent, respectively. As will be appreciated, other gradient schemes, e.g., full color, may be used.

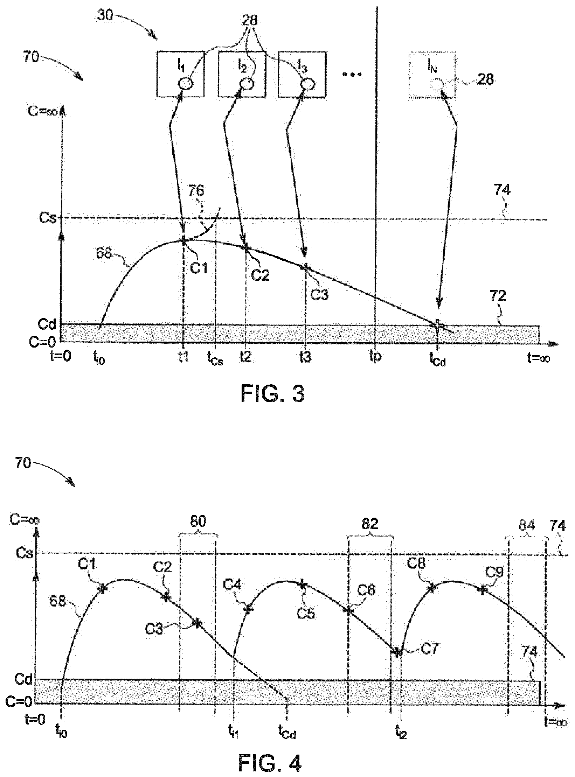

Moving to FIG. 3, a kinetic model 70 is then applied to the contrast data obtained from each image 30 to calculate a measured/estimated amount of the contrast agent within the ROI 28 for each of the one or more images 30. As will be appreciated, the kinetic model 70 may be based at least in part on fluid kinetics such that the kinetic model 70 is able to model the flux, i.e., volume per unit of time, of the contrast agent within the patient 12

The kinetic model 70 then generates a predictive curve (represented by the solid line 68) of the amount C of the contrast agent within the ROI based at least in part on the measured/estimated amount of the contrast agent for each of the one or more images 30. As will be appreciated, in embodiments, the predictive curve 68 indicates the amount C of the contrast agent within the patient 12 over a period of time t. For example, shown in FIG. 3 is an embodiment in which the controller 22 obtains three images I₁, I₂, and I₃ at times t₁, t₂, and t₃, having measured/estimated contrast amounts of C₁, C₂, and C₃, respectively, subsequent to an injection of the contrast agent into the patient 12 at time tᵢ₀. The kinetic model 70 then generates the shown predictive curve 68 based at least in part on the values of C₁, C₂, and C₃. In other words, the kinetic model 70 is used to fit the contrast data, e.g., C₁, C₂, and C₃, to the predictive curve 68. As will be appreciated, the kinetic model 70 may utilize/incorporate additional parameters and/or constraints to generate the predictive curve 68. For example, in embodiments, the kinetic model 70 may be based at least in part on a volume of the patient 12, a weight of the patient 12, a mass of the patient 12, a morphology of the patient 12, and/or historical data of the contrast agent within the patient 12 and/or a sample population.

Accordingly, in embodiments, the kinetic model 70 may calculate/estimate a contrast agent decay time t_{Cd}, which represents the time when the amount of the contrast agent within the patient 12 is predicted to drop below/exceed a lower contrast agent threshold 72. The lower contrast agent threshold 72 may correspond to an amount C_{d} of the contrast agent within the patient 12 that is insufficient to maintain a desired image quality in an image Iₙ that has yet to be acquired, i.e., I₁, I₂, I₃ are acquired prior to the present time tₚ, while Iₙ is acquired after tₚ. Similarly, the kinetic model 70 may calculate/estimate a contrast agent saturation time t_{Cs}, which represents the time when the amount of the contrast agent within the patient 12 is predicted to rise above/exceed an upper contrast agent threshold 74, as shown by the dashed line 76. In embodiments, the upper contrast agent threshold 74 may correspond to an amount Cₛ that poses a significant risk to the patient 12, e.g., organ failure. Thus, as will be understood, the dashed segment 76 in FIG. 3 represents a hypothetical path of the predictive curve 68 in a scenario where too much of the contrast agent was injected into the patient at time tᵢ₀. Further, while the predictive curve 68 is shown herein as a continuous line, it will be understood that, in embodiments, the predictive curve 68 may be broken and/or have a shape other than a curve, e.g., rectangular, triangular, and/or any other shape that models the decay of the contrast agent.

Turning now to FIG. 4, in certain aspects, the kinetic model 70 may calculate/generate one or more injection times, e.g., tᵢ₁, tᵢ₂, etc, for the contrast agent via the kinetic model 70 based at least in part on the predictive curve 68. As will be appreciated, in embodiments, the one or more injection times tn, tᵢ₂ may be configured to prevent the amount of the contrast agent within the patient 12 from exceeding the upper contrast agent threshold 74 and/or the lower contrast agent threshold 72. In embodiments, the kinetic model 70 may also calculate one or more injection parameters such as duration, volume of contrast agent to be injected, etc.

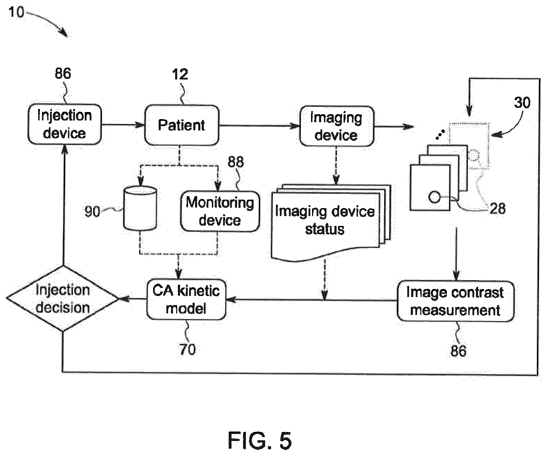

As will be appreciated, the controller 22 may generate an announcement via an announcer 78 (FIG. 1) that notifies an operator of the system 10 that an injection time tᵢ₁ is approaching, is occurring, and/or has occurred. Similarly, the controller 22 may generate an announcement via the announcer 78 prior to at least one of the contrast saturation time t_{Cs} and the contrast agent decay time t_{Cd}. As will be understood, the announcer 78 may be an optical device, auditory device, and/or any other device that is capable of conveying information to the operator of the system 10. Accordingly, in embodiments, the controller 22 may generate an announcement during one or more warning windows 80, 82, 84 during which an amount of the contrast agent should be injected into the patient 12 in order to avoid exceeding the lower contrast agent threshold 74. Further, in embodiments, the controller 22 may also inject an additional amount of the contrast agent into the patient 12 via an injector 86 (FIGS. 2 and 5) in accordance with one or more of the aforementioned injection parameters calculated via the kinetic model 70.

Referring now to FIGS. 4 and 5, in embodiments, the kinetic model 70 may dynamically adjust the predictive curve 68 while obtaining the contrast data from each image of the one or more images 30. For example, the controller 22 may obtain a first image I₁ after an initial injection of the contrast agent into the patient 12 at tie and determine/estimate a first amount C₁ of the contrast agent within the ROI 28. Utilizing the kinetic model 70, the controller 22 may then calculate an initial value for tᵢ₁ based on C₁. The controller 22 may then obtain a second image I₂ and determine/estimate C₂ of the contrast agent within the ROI 28. Based on the information from C₁ and C₂, the kinetic model 70 may update/adjust the shape of the predictive curve 68 such that tᵢ₁, t_{Cd}, and/or the warning window 80 shift in time. The controller 22 may then obtain a third image I₃ and determine/estimate C₃ of the contrast agent within the ROI 28, and the kinetic model 70 may again update/adjust the shape of the predictive curve 68 based on the information from C₁, C₂, and C₃. As will be appreciated, the more images 30 acquired between an injection of the contrast agent and the point at which the amount of the contrast agent within the patient 12 actually exceeds a threshold 72, 74, the more accurate the predictive curve 68 becomes. In other words, in embodiments, increasing the number of images 30 between injections of the contrast agent increases the resolution/accuracy of the predictive model 70.

Additionally, in embodiments, the kinetic model 70 may be able to determine the type of the contrast agent based on analyzing the predictive curve 68 and comparing it to one or more known decay curves for one or more known contrast agents.

As will be understood, the resolution/accuracy of the kinetic model 70 may also be increased by incorporating information about the patient 12, e.g., their weight, volume, mass, blood pressure, respiratory rate, and/or any other factor which may influence the flow and/or filtration of the contrast agent within the patient 12, which may be gathered via a patient monitoring device 88, e.g., medical sensors to include an oximeter. Further, historical data stored in a database 90 may also increase the resolution/accuracy of the kinetic model 70. For example, the historical data may include a previously calculated and/or measured predictive curve of the contrast agent within the patient 12 and/or within a sample population, which in turn may be used as a baseline by the kinetic model 70 to generate the current predictive curve 68. Further still, in embodiments, the kinetic model 70 may adjust/update the aforementioned injection parameters as the predictive curve 68 is updated.

Finally, it is also to be understood that the system 10 may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system may include at least one processor and system memory / data storage structures, which may include random access memory (RAM) and read-only memory (ROM). The at least one processor of the system may include one or more conventional microprocessors and one or more supplementary co-processors such as math co-processors or the like. The data storage structures discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. The term "computer-readable medium," as used herein, refers to any medium that provides or participates in providing instructions to the at least one processor of the system 10 (or any other processor of a device described herein) for execution. Such a medium may take many forms, including but not limited to, non-volatile media and volatile media. Non-volatile media include, for example, optical, magnetic, or opto-magnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is further to be understood that the above description is intended to be illustrative, and not restrictive. For example, the above-described embodiments (and/or aspects thereof) may be used in combination with each other. Additionally, many modifications may be made to adapt a particular situation or material to the teachings of the invention without departing from its scope.

For example, in an embodiment, a method for monitoring an amount of a contrast agent within an object is provided. The method includes obtaining contrast data from one or more images of an object via an imaging device. The contrast data corresponds to the contrast agent. The method further includes calculating a measured amount of the contrast agent for each of the one or more images by applying a kinetic model to the contrast data, and generating a predictive curve of the amount of the contrast agent via the kinetic model. The kinetic model generates the predictive curve based at least in part on the measured amount of the contrast agent for each of the one or more images. In certain embodiments, the method further includes calculating one or more injection times for the contrast agent via the kinetic model based at least in part on the predictive curve. In certain embodiments, the method further includes injecting an additional amount of the contrast agent into the object at each of the one or more injection times. In certain embodiments, the one or more injection times are configured to prevent the amount of the contrast agent within the object from exceeding at least one of an upper contrast agent threshold and a lower contrast agent threshold. In certain embodiments, the method further includes calculating at least one of a contrast agent saturation time and a contrast agent decay time, and generating an announcement prior to at least one of the contrast agent saturation time and the contrast agent decay time. In certain embodiments, the kinetic model dynamically adjusts the predictive curve while obtaining the contrast data from each image of the one or more images. In certain embodiments, the kinetic model is based at least in part on one or more of a volume of the object, a weight of the object, a mass of the object, a morphology of the object, and historical data of the contrast agent within the object. In certain embodiments, the method further includes determining a type of the contrast agent based at least in part on the contrast data via the kinetic model. In certain embodiments, the one or more images of the object are obtained during a breast biopsy procedure.

Other embodiments provide for a system for monitoring an amount of a contrast agent within an object. The system includes a controller in electronic communication with an imaging device and operative to obtain contrast data from one or more images of the object via the imaging device. The contrast data corresponds to the contrast agent. The controller is further operative to calculate a measured amount of the contrast agent for each of the one or more images by applying a kinetic model to the contrast data, and to generate a predictive curve of the amount of the contrast agent via the kinetic model. The kinetic model generates the predictive curve based at least in part on the measured amount of the contrast agent for each of the one or more images. In certain embodiments, the controller is further operative to calculate one or more injection times for the contrast agent via the kinetic model based at least in part on the predictive curve. In certain embodiments, the system further includes an injection device in electronic communication with the controller. In such embodiments, the controller is further operative to inject an additional amount of the contrast agent into the object at each of the one or more injection times via the injection device. In certain embodiments, the one or more injection times are configured to prevent the amount of the contrast agent within the object from exceeding at least one of an upper contrast agent threshold and a lower contrast agent threshold. In certain embodiments, the system further includes an announcer in electronic communication with the controller. In such embodiments, the controller is further operative to calculate at least one of a contrast agent saturation time and a contrast agent decay time; and to generate an announcement prior to at least one of the contrast agent saturation time and the contrast agent decay time via the announcer. In certain embodiments, the kinetic model dynamically adjusts the predictive curve while the controller obtains the contrast data from each image of the one or more images. In certain embodiments, the kinetic model is based at least in part on one or more of a volume of the object, a weight of the object, a mass of the object, a morphology of the object, and historical data of the contrast agent within the object. In certain embodiments, the controller is further operative to determine a type of the contrast agent based at least in part on the contrast data via the kinetic model. In certain embodiments, the imaging device forms part of a breast biopsy apparatus.

Yet still other embodiments provide for a non-transitory computer readable medium storing instructions. The stored instructions are configured to adapt a controller to obtain contrast data from one or more images of an object via the imaging device. The contrast data corresponds to the contrast agent. The stored instructions are further configured to calculate a measured amount of the contrast agent for each of the one or more images by applying a kinetic model to the contrast data, and to generate a predictive curve of the amount of the contrast agent via the kinetic model. The kinetic model generates the predictive curve based at least in part on the measured amount of the contrast agent for each of the one or more images. In certain embodiments, the stored instructions are further configured to adapt the controller to calculate one or more injection times for the contrast agent via the kinetic model based at least in part on the predictive curve.

Accordingly, as will be appreciated, by generating a predictive curve of a contrast agent based on acquired images, some embodiments of the invention provide for more accurate monitoring of the amount and/or control over the flux of the contrast agent within a patient. Thus, some embodiments of the present invention may reduce the total amount of contrast agent injected into a patient during a medical imaging procedure, which in turn may reduce the risk of organ failure while maintaining and/or improving image quality.

Further, and as will be appreciated, some embodiments of the present invention provide for a framework to control contrast agent flux, which in turn may optimize the clinical workflow efficiency and/or the visibility of lesions during a CESM-guided biopsy procedure.

The scope of the invention should be determined with reference to the appended claims.

## Claims

1. A system (10) for monitoring an amount of a contrast agent within a patient (12), the patient (12) having been given a first injection (52) of the contrast agent at time tᵢ₀ at a region of interest, ROI, (28) of the patient (12), the system (10) comprising:
an imaging device (18, 20) configured to obtain a plurality of images (30, I_{1...n}) of the ROI (28) at a plurality of times (t_{1...n});
a controller (22) in electronic communication with the imaging device (18, 20) and operative to:
obtain contrast data from the plurality of images (30, I_{1...n}), the contrast data corresponding to the contrast agent, wherein the contrast data provides an indication of the amount of the contrast agent within the patient (12);
calculate an estimated amount of the contrast agent (C_{1...n}) within the ROI (28) for each of the plurality of images (30, I_{1...n}) by applying a kinetic model (70) to the contrast data, wherein the kinetic model (70) is based at least in part on fluid kinetics such that the kinetic model (70) is able to model a flux of the contrast agent within the patient (12);
generate a predictive curve (68) of the amount of the contrast agent (C_{1...n}) within the ROI (28) via the kinetic model (70);
wherein the kinetic model (70) generates the predictive curve (68) by fitting the estimated amount of the contrast agent (C_{1...n}) within the ROI (28) for each of the plurality of images (30) to a curve modelling the decay of the contrast agent; and
calculate a plurality of injection times (t_{i1...in}) for the contrast agent via the kinetic model (70) based at least in part on the predictive curve (68), wherein the plurality of injection times (t_{i1...in}) are configured to prevent the amount of the contrast agent within the patient (12) from at least one of exceeding an upper contrast agent threshold (74) and dropping below a lower contrast agent threshold (72); and
an injection device (86) in electronic communication with the controller (22),
wherein the controller (22) is further operative to:
inject an additional amount of the contrast agent into the patient (12) at each of the plurality of injection times (t_{i1...in}) via the injection device (86).

2. The system (10) of claim 1 further comprising:
an announcer (78) in electronic communication with the controller (22); and
wherein the controller (22) is further operative to:
calculate at least one of a contrast agent saturation time (t_{Cs}) and a contrast agent decay time (t_{Cd}); and
generate an announcement prior to at least one of the contrast agent saturation time (t_{Cs}) and the contrast agent decay time (t_{Cd}) via the announcer (78).

3. The system (10) of any of claims 1 to 2, wherein the kinetic model (70) dynamically adjusts the predictive curve (68) while the controller (22) obtains the contrast data from each image of the plurality of images (30, I_{1...n}).

4. The system (10) of any preceding claim, wherein the kinetic model (70) is based at least in part on one or more of a volume of the patient (12), a weight of the patient (12), a mass of the patient (12), a morphology of the patient (12), and historical data of the contrast agent within the patient (12).

5. The system (10) of any preceding claim, wherein the controller is further configured to:
determine a type of the contrast agent based at least in part on the contrast data via the kinetic model (70).

6. The system (10) of any preceding claim, wherein the one or more images (30, I_{1...n}) of the patient (12) are obtained during a breast biopsy procedure.

7. A computer readable medium comprising instructions for a controller of a system for monitoring an amount of a contrast agent within a patient (12), the patient (12) having been given a first injection (52) of the contrast agent at time tᵢ₀ at a region of interest, ROI, (28) of the patient (12), the instructions, when executed, causing the controller to:
obtain a plurality of images (30, I_{1...n}) of the ROI (28) at a plurality of times (t_{1...n}) via an imaging device (18, 20);
obtain contrast data from the plurality of images (30, I_{1...n}), the contrast data corresponding to the contrast agent, wherein the contrast data provides an indication of the amount of the contrast agent within the patient (12);
calculate an estimated amount of the contrast agent (C_{1...n}) within the ROI (28) for each of the plurality of images (30, I_{1...n}) by applying a kinetic model (70) to the contrast data, wherein the kinetic model (70) is based at least in part on fluid kinetics such that the kinetic model (70) is able to model a flux of the contrast agent within the patient (12);
generate a predictive curve (68) of the amount of the contrast agent (C_{1...n}) within the ROI (28) via the kinetic model (70);
wherein the kinetic model (70) generates the predictive curve (68) by fitting the estimated amount of the contrast agent (C_{1...n}) within the ROI (28) for each of the plurality of images (30) to a curve modelling the decay of the contrast agent;
calculate a plurality of injection times (t_{i1...in}) for the contrast agent via the kinetic model (70) based at least in part on the predictive curve (68), wherein the plurality of injection times (t_{i1...in}) are configured to prevent the amount of the contrast agent within the patient (12) from at least one of exceeding an upper contrast agent threshold (74) and dropping below a lower contrast agent threshold (72); and
inject an additional amount of the contrast agent into the patient (12) at each of the plurality of injection times (t_{i1...in}) via an injection device (86) in electronic communication with the controller.

8. The computer readable medium of claim 7, the instructions, when executed, further causing the controller to:
calculate at least one of a contrast agent saturation time (t_{Cs}) and a contrast agent decay time (t_{Cd}); and
generate an announcement prior to at least one of the contrast agent saturation time (t_{Cs}) and the contrast agent decay time (t_{Cd}) via an announcer (78) in electronic communication with the controller (22).

9. The computer readable medium of any of claims 7 to 8, wherein the kinetic model (70) dynamically adjusts the predictive curve (68) while the controller (22) obtains the contrast data from each image of the plurality of images (30, I_{1...n}).

10. The computer readable medium of any of claims 7-9, wherein the kinetic model (70) is based at least in part on one or more of a volume of the patient (12), a weight of the patient (12), a mass of the patient (12), a morphology of the patient (12), and historical data of the contrast agent within the patient (12).

11. The computer readable medium of any of claims 7-10, the instructions, when executed, further causing the controller to:
determine a type of the contrast agent based at least in part on the contrast data via the kinetic model (70).

12. The computer readable medium of any of claims 7-11, wherein the one or more images (30, I_{1...n}) of the patient (12) are obtained during a breast biopsy procedure.

## Patentansprüche

1. System (10) zum Überwachen einer Menge eines Kontrastmittels in einem Patienten (12), wobei der Patient (12) eine erste Injektion (52) des Kontrastmittels zum Zeitpunkt tᵢ₀ in einer Region von Interesse, ROI, (28) des Patienten (12) erhalten hat, wobei das System (10) Folgendes umfasst:
eine Bildgebungsvorrichtung (18, 20), die dazu ausgelegt ist, eine Vielzahl von Bildern (30, I_{1...n}) der ROI (28) zu einer Vielzahl von Zeitpunkten (t_{1...n}) zu erhalten;
eine Steuervorrichtung (22) in elektronischer Kommunikation mit der Bildgebungsvorrichtung (18, 20) und dazu betreibbar:
Kontrastdaten aus der Vielzahl von Bildern (30, I_{1...n}) zu erhalten, wobei die Kontrastdaten dem Kontrastmittel entsprechen, wobei die Kontrastdaten eine Angabe über die Menge des Kontrastmittels im Patienten (12) bereitstellen;
eine geschätzte Menge des Kontrastmittels (C_{1...n}) innerhalb der ROI (28) für jedes der Vielzahl von Bildern (30, I_{1...n}) durch Anwenden eines kinetischen Modells (70) auf die Kontrastdaten zu berechnen, wobei das kinetische Modell (70) zumindest teilweise auf Fluidkinetik basiert, sodass das kinetische Modell (70) einen Fluss des Kontrastmittels innerhalb des Patienten (12) modellieren kann;
eine Vorhersagekurve (68) für die Menge des Kontrastmittels (C_{1...n}) innerhalb der ROI (28) über das kinetische Modell (70) zu erzeugen;
wobei das kinetische Modell (70) die Vorhersagekurve (68) erzeugt, indem es die geschätzte Menge des Kontrastmittels (C_{1...n}) innerhalb der ROI (28) für jedes der Vielzahl von Bildern (30) an eine Kurve anpasst, die das Abklingen des Kontrastmittels modelliert; und
Berechnen einer Vielzahl von Injektionszeitpunkten (t_{i1...in}) für das Kontrastmittel über das kinetische Modell (70) basierend zumindest teilweise auf der Vorhersagekurve (68), wobei die Vielzahl von Injektionszeitpunkten (t_{i1...in}) dazu ausgelegt sind, zu verhindern, dass die Menge des Kontrastmittels im Patienten (12) zumindest einen oberen Kontrastmittelschwellenwert (74) überschreitet und einen unteren Kontrastmittelschwellenwert (72) unterschreitet; und
eine Injektionsvorrichtung (86) in elektronischer Kommunikation mit der Steuervorrichtung (22),
wobei die Steuervorrichtung (22) ferner dazu betreibbar ist:
zu jedem der Vielzahl von Injektionszeitpunkten (t_{i1...in}) über die Injektionsvorrichtung (86) eine zusätzliche Menge des Kontrastmittels in den Patienten (12) zu injizieren.

2. Verfahren (10) gemäß Anspruch 1, ferner Folgendes umfassend:
einen Ankündiger (78) in elektronischer Kommunikation mit der Steuervorrichtung (22); und
wobei die Steuervorrichtung (22) ferner dazu betreibbar ist:
mindestens eines einer Kontrastmittelsättigungszeit (t_{Cs}) und einer Kontrastmittelabklingzeit (t_{Cd}) zu berechnen; und
vor mindestens eines der Kontrastmittelsättigungszeit (t_{Cs}) und der Kontrastmittelabklingzeit (t_{Cd}) eine Ankündigung über den Ankündiger (78) erzeugen.

3. System (10) gemäß einem der Ansprüche 1 bis 2, wobei das kinetische Modell (70) die Vorhersagekurve (68) dynamisch anpasst, während die Steuervorrichtung (22) die Kontrastdaten aus jedem Bild der Vielzahl von Bildern (30, I_{1...n}) erhält.

4. System (10) gemäß einem der vorhergehenden Ansprüche, wobei das kinetische Modell (70) mindestens teilweise auf einem oder mehreren eines Volumens des Patienten (12), eines Gewichts des Patienten (12), einer Masse des Patienten (12), einer Morphologie des Patienten (12) und historischen Daten des Kontrastmittels im Patienten (12) basiert.

5. System (10) gemäß einem der vorhergehenden Ansprüche, wobei die Steuervorrichtung ferner dazu ausgelegt ist:
eine Art des Kontrastmittels mindestens teilweise basierend auf den Kontrastdaten über das kinetische Modell (70) zu bestimmen.

6. System (10) gemäß einem der vorhergehenden Ansprüche, wobei das eine oder die mehreren Bilder (30, I_{1...n}) einer Patientin (12) während eines Brustbiopsieverfahrens erhalten werden.

7. Computerlesbares Medium, umfassend Anweisungen für eine Steuervorrichtung eines Systems zum Überwachen einer Menge eines Kontrastmittels in einem Patienten (12), wobei der Patient (12) eine erste Injektion (52) des Kontrastmittels zum Zeitpunkt tᵢ₀ in einer Region von Interesse, ROI, (28) des Patienten (12) erhalten hat, wobei die Anweisungen beim Ausführen die Steuervorrichtung dazu veranlassen:
eine Vielzahl von Bildern (30, I_{1...n}) der ROI (28) zu einer Vielzahl von Zeitpunkten (t_{1...n}) über eine Bildgebungsvorrichtung (18, 20) zu erhalten;
Kontrastdaten aus der Vielzahl von Bildern (30, I_{1...n}) zu erhalten, wobei die Kontrastdaten dem Kontrastmittel entsprechen, wobei die Kontrastdaten eine Angabe über die Menge des Kontrastmittels im Patienten (12) bereitstellen;
eine geschätzte Menge des Kontrastmittels (C_{1...n}) innerhalb der ROI (28) für jedes der Vielzahl von Bildern (30, I_{1...n}) durch Anwenden eines kinetischen Modells (70) auf die Kontrastdaten zu berechnen, wobei das kinetische Modell (70) zumindest teilweise auf Fluidkinetik basiert, sodass das kinetische Modell (70) einen Fluss des Kontrastmittels innerhalb des Patienten (12) modellieren kann;
eine Vorhersagekurve (68) für die Menge des Kontrastmittels (C_{1...n}) innerhalb der ROI (28) über das kinetische Modell (70) zu erzeugen;
wobei das kinetische Modell (70) die Vorhersagekurve (68) durch Anpassen der geschätzten Menge des Kontrastmittels (C_{1...n}) innerhalb der ROI (28) für jedes der Vielzahl von Bildern (30) an eine Kurve erzeugt, die das Abklingen des Kontrastmittels modelliert;
Berechnen einer Vielzahl von Injektionszeitpunkten (t_{i1...in}) für das Kontrastmittel über das kinetische Modell (70) basierend zumindest teilweise auf der Vorhersagekurve (68), wobei die Vielzahl von Injektionszeitpunkten (t_{i1...in}) dazu ausgelegt sind, zu verhindern, dass die Menge des Kontrastmittels im Patienten (12) zumindest einen oberen Kontrastmittelschwellenwert (74) überschreitet und einen unteren Kontrastmittelschwellenwert (72) unterschreitet; und
zu jedem der Vielzahl von Injektionszeitpunkten (t_{i1...in}) eine zusätzliche Menge des Kontrastmittels über eine Injektionsvorrichtung (86) in elektronischer Kommunikation mit der Steuervorrichtung in den Patienten (12) injizieren.

8. Computerlesbares Medium gemäß Anspruch 7, wobei die Anweisungen beim Ausführen ferner die Steuervorrichtung dazu veranlassen:
mindestens eines einer Kontrastmittelsättigungszeit (t_{Cs}) und einer Kontrastmittelabklingzeit (t_{Cd}) zu berechnen; und
eine Ankündigung vor mindestens einer der Kontrastmittelsättigungszeit (t_{Cs}) und der Kontrastmittelabklingzeit (t_{Cd}) über einen Ankündiger (78) in elektronischer Kommunikation mit der Steuervorrichtung (22) zu erzeugen.

9. Computerlesbares Medium gemäß einem der Ansprüche 7 bis 8, wobei das kinetische Modell (70) die Vorhersagekurve (68) dynamisch anpasst, während die Steuervorrichtung (22) die Kontrastdaten aus jedem Bild der Vielzahl von Bildern (30, I_{1...n}) erhält.

10. Computerlesbares Medium gemäß einem der Ansprüche 7-9, wobei das kinetische Modell (70) mindestens teilweise auf einem oder mehreren eines Volumens des Patienten (12), eines Gewichts des Patienten (12), einer Masse des Patienten (12), einer Morphologie des Patienten (12) und historischen Daten des Kontrastmittels im Patienten (12) basiert.

11. Computerlesbares Medium gemäß einem der Ansprüche 7-10, wobei die Anweisungen beim Ausführen ferner die Steuervorrichtung dazu veranlassen:
eine Art des Kontrastmittels mindestens teilweise basierend auf den Kontrastdaten über das kinetische Modell (70) zu bestimmen.

12. Computerlesbares Medium gemäß einem der Ansprüche 7-11, wobei das eine oder die mehreren Bilder (30, I_{1...n}) einer Patientin (12) während eines Brustbiopsieverfahrens erhalten werden.

## Revendications

1. Système (10) de surveillance d'une quantité d'un agent de contraste chez un patient (12), le patient (12) ayant reçu une première injection (52) de l'agent de contraste à l'instant tᵢ₀ au niveau d'une région d'intérêt, ROI, (28) du patient (12), le système (10) comprenant :
un dispositif d'imagerie (18, 20) configuré pour obtenir une pluralité d'images (30, I_{1...n}) de la ROI (28) à une pluralité d'instants (t_{1...n}) ;
un contrôleur (22) en communication électronique avec le dispositif d'imagerie (18, 20) et apte à :
obtenir des données de contraste à partir de la pluralité d'images (30, I_{1...n}), les données de contraste correspondant à l'agent de contraste, les données de contraste fournissant une indication de la quantité de l'agent de contraste chez le patient (12) ;
calculer une quantité estimée de l'agent de contraste (C_{1...n}) au sein de la ROI (28) pour chacune de la pluralité d'images (30, I_{1...n}) en appliquant un modèle cinétique (70) aux données de contraste, le modèle cinétique (70) étant basé au moins en partie sur la cinétique des fluides de façon à permettre au modèle cinétique (70) de modéliser un flux de l'agent de contraste chez le patient (12) ;
générer une courbe prédictive (68) de la quantité de l'agent de contraste (C_{1...n}) au sein de la ROI (28) via le modèle cinétique (70) ;
le modèle cinétique (70) générant la courbe prédictive (68) en ajustant la quantité estimée de l'agent de contraste (C_{1...n}) au sein de la ROI (28) pour chacune de la pluralité d'images (30) sur une courbe modélisant la décroissance de l'agent de contraste ; et
calculer une pluralité d'instants d'injection (t_{i1...in}) pour l'agent de contraste via le modèle cinétique (70) sur la base au moins en partie de la courbe prédictive (68), la pluralité d'instants d'injection (t_{i1...in}) étant configurés pour empêcher la quantité de l'agent de contraste chez le patient (12) de dépasser un seuil supérieur (74) d'agent de contraste et/ou de passer au-dessous d'un seuil inférieur (72) d'agent de contraste ; et
un dispositif d'injection (86) en communication électronique avec le contrôleur (22),
le contrôleur (22) étant apte en outre à :
injecter une quantité additionnelle de l'agent de contraste dans le patient (12) à chacun de la pluralité d'instants d'injection (t_{i1...in}) via le dispositif d'injection (86).

2. Système (10) selon la revendication 1, comprenant en outre :
un annonceur (78) en communication électronique avec le contrôleur (22) ; et
le contrôleur (22) étant apte en outre à :
calculer un temps de saturation (t_{Cs}) d'agent de contraste et/ou un temps de décroissance (t_{Cd}) d'agent de contraste ; et
générer une annonce préalablement au temps de saturation (t_{Cs}) d'agent de contraste et/ou au temps de décroissance (t_{Cd}) d'agent de contraste via l'annonceur (78) .

3. Système (10) selon l'une quelconque des revendications 1 à 2, dans lequel le modèle cinétique (70) ajuste dynamiquement la courbe prédictive (68) tandis que le contrôleur (22) obtient les données de contraste à partir de chaque image de la pluralité d'images (30, I_{1...n}).

4. Système (10) selon l'une quelconque des revendications précédentes, dans lequel le modèle cinétique (70) est basé au moins en partie sur un volume du patient (12) et/ou un poids du patient (12) et/ou une masse du patient (12) et/ou une morphologie du patient (12) et/ou des données historiques de l'agent de contraste chez le patient (12).

5. Système (10) selon l'une quelconque des revendications précédentes, dans lequel le contrôleur est configuré en outre pour :
déterminer un type de l'agent de contraste sur la base au moins en partie des données de contraste via le modèle cinétique (70).

6. Système (10) selon l'une quelconque des revendications précédentes, dans lequel les une ou plusieurs images (30, I_{1...n}) du patient (12) sont obtenues au cours d'un acte de biopsie mammaire.

7. Support lisible par ordinateur comprenant des instructions pour un contrôleur d'un système de surveillance d'une quantité d'un agent de contraste chez un patient (12), le patient (12) ayant reçu une première injection (52) de l'agent de contraste à l'instant tᵢ₀ au niveau d'une région d'intérêt, ROI, (28) du patient (12), les instructions, lorsqu'elles sont exécutées, amenant le contrôleur à :
obtenir une pluralité d'images (30, I_{1...n}) de la ROI (28) à une pluralité d'instants (t_{1...n}) via un dispositif d'imagerie (18, 20) ;
obtenir des données de contraste à partir de la pluralité d'images (30, I_{1...n}), les données de contraste correspondant à l'agent de contraste, les données de contraste fournissant une indication de la quantité de l'agent de contraste chez le patient (12) ;
calculer une quantité estimée de l'agent de contraste (C_{1...n}) au sein de la ROI (28) pour chacune de la pluralité d'images (30, I_{1...n}) en appliquant un modèle cinétique (70) aux données de contraste, le modèle cinétique (70) étant basé au moins en partie sur la cinétique des fluides de façon à permettre au modèle cinétique (70) de modéliser un flux de l'agent de contraste chez le patient (12) ;
générer une courbe prédictive (68) de la quantité de l'agent de contraste (C_{1...n}) au sein de la ROI (28) via le modèle cinétique (70) ;
le modèle cinétique (70) générant la courbe prédictive (68) en ajustant la quantité estimée de l'agent de contraste (C_{1...n}) au sein de la ROI (28) pour chacune de la pluralité d'images (30) sur une courbe modélisant la décroissance de l'agent de contraste ;
calculer une pluralité d'instants d'injection (t_{i1...in}) pour l'agent de contraste via le modèle cinétique (70) sur la base au moins en partie de la courbe prédictive (68), la pluralité d'instants d'injection (t_{i1...in}) étant configurés pour empêcher la quantité de l'agent de contraste chez le patient (12) de dépasser un seuil supérieur (74) d'agent de contraste et/ou de passer au-dessous d'un seuil inférieur (72) d'agent de contraste ; et
injecter une quantité additionnelle de l'agent de contraste dans le patient (12) à chacun de la pluralité d'instants d'injection (t_{i1...in}) via un dispositif d'injection (86) en communication électronique avec le contrôleur.

8. Support lisible par ordinateur selon la revendication 7, les instructions, lorsqu'elles sont exécutées, amenant en outre le contrôleur à :
calculer un temps de saturation (t_{Cs}) d'agent de contraste et/ou un temps de décroissance (t_{Cd}) d'agent de contraste ; et
générer une annonce préalablement au temps de saturation (t_{Cs}) d'agent de contraste et/ou au temps de décroissance (t_{Cd}) d'agent de contraste via un annonceur (78) en communication électronique avec le contrôleur (22).

9. Support lisible par ordinateur selon l'une quelconque des revendications 7 à 8, dans lequel le modèle cinétique (70) ajuste dynamiquement la courbe prédictive (68) tandis que le contrôleur (22) obtient les données de contraste à partir de chaque image de la pluralité d'images (30, I_{1...n}).

10. Support lisible par ordinateur selon l'une quelconque des revendications 7 à 9, dans lequel le modèle cinétique (70) est basé au moins en partie sur un volume du patient (12) et/ou un poids du patient (12) et/ou une masse du patient (12) et/ou une morphologie du patient (12) et/ou des données historiques de l'agent de contraste chez le patient (12).

11. Support lisible par ordinateur selon l'une quelconque des revendications 7 à 10, les instructions, lorsqu'elles sont exécutées, amenant en outre le contrôleur à :
déterminer un type de l'agent de contraste sur la base au moins en partie des données de contraste via le modèle cinétique (70).

12. Support lisible par ordinateur selon l'une quelconque des revendications 7 à 11, dans lequel les une ou plusieurs images (30, I_{1...n}) du patient (12) sont obtenues au cours d'un acte de biopsie mammaire.
